Europäisches Patentamt

European Patent Office

Office européen des brevets

(19)

(11) Numéro de publication: **0 145 589**
**A2**

(12) DEMANDE DE BREVET EUROPEEN

(21) Numéro de dépôt: **84402467.9**

(22) Date de dépôt: **30.11.84**

(51) Int. Cl.⁴: **C 12 N 15/00,** C 12 N 5/00, C 12 P 21/02

(30) Priorité: **30.11.83 FR 8319168**

(43) Date de publication de la demande: **19.06.85 Bulletin 85/25**

(84) Etats contractants désignés: **AT BE CH DE GB IT LI LU NL SE**

(71) Demandeur: **INSTITUT PASTEUR Fondation reconnue d'utilité publique, 28 rue du Docteur Roux, F-75724 Paris Cédex 15 (FR)**

(72) Inventeur: **Garapin, Florence, 23, rue Gastagnary, F-75015 Paris (FR)**
Inventeur: **Garapin, Axel, 23, rue Gastagnary, F-75015 Paris (FR)**
Inventeur: **Horaud, Florian, 2, rue Albert de Mun, F-92190 Meudon (FR)**

(74) Mandataire: **Gutmann, Ernest et al, Cabinet Plasseraud 84, rue d'Amsterdam, F-75009 Paris (FR)**

(54) **Culture de cellules eucaryotes productrices d'une protéine vaccinante, notamment d'antigène HBs.**

(57) L'invention concerne des cellules Vero productrices d'un polypeptide immunogène ayant les propriétés immunologiques de l'antigène HBs. Elles sont constituées par des cellules Vero transformées par un plasmide porteur d'une séquence codant pour ledit polypeptide, sans que soit modifiée la capacité de ces cellules Vero d'être reconnues par des anticorps spécifiques anti-Vero et sans que soit affectée leur capacité d'être infectées par des poliovirus.

EP 0 145 589 A2

1

Culture de cellules eucaryotes productrices d'une protéine vaccinante, notamment d'antigène HBs.

————— — —

L'invention concerne une culture cellulaire provenant d'organismes de mammifères, notamment de singe, et rendue apte à secréter de façon stable et continue un polypeptide ayant les propriétés immunogènes de l'antigène HBs. Elle concerne plus particulièrement encore une culture cellulaire présentant les propriétés sus-indiquées en raison du caractère transformé des cellules qui la composent, par un plasmide contenant à la fois un insérat constitué par un ADN codant pour ledit polypeptide immunogène et un marqueur du type décrit dans la demande de brevet principal n° 81 04137 du 2 mars 1981.

Il est rappelé que la demande de brevet principal décrit entre autres des cultures de cellules eucaryotes d'organismes supérieurs, notamment originaires de mammifères, et appropriées à la fabrication de vaccins, ces cellules étant caractérisées en ce qu'elles contiennent, à titre de marqueur génétique interne sélectif, un ADN, circulaire ou non, contenant un promoteur eucaryote ou reconnu par sa capacité à diriger l'expression dans une cellule eucaryote, de préférence d'un organisme supérieur, notamment de mammifère, d'un gène qui lui est normalement associé, et une séquence d'ADN codant pour une enzyme susceptible d'inactiver un antibiotique, capable de pénétrer dans au moins certaines cellules eucaryotes ou de les détruire par un processus toxique ou les deux à la fois, cette séquence étant liée au promoteur susdit et sous son contrôle direct.

0145589

2

Un marqueur préféré est celui dans lequel la séquence d'ADN codant pour une enzyme susceptible d'inactiver un antibiotique est choisie parmi celles qui codent une enzyme capable d'inactiver l'antibiotique G 418.

De préférence encore, le plasmide responsable du caractère transformé des cellules susdites, contient également la séquence d'ADN codant pour le peptide immunogène particulier dont la production est recherchée. Il a été indiqué dans la demande de brevet français que cette dernière séquence d'ADN était avantageusement constituée par tout ou partie du gène S contenu dans le génome du virus de l'hépatite B. Un exemple de construction du plasmide utilisable pour la transformation de cellules de mammifères, notamment de singe, a encore été décrit dans la demande de brevet européen n° 82 900640 déposée le 2 mars 1983. Le plasmide obtenu a été dénommé pAG 66. Des cellules transformées, et plus particulièrement des cellules Vero transformées par ce plasmide, ont pu être détectées grâce à la capacité que leur confère le marqueur de se développer dans des milieux de culture contenant même l'antibiotique G 418. Les mêmes cellules se sont révélées capables de secréter un peptide immunogène présentant les propriétés immunologiques de l'antigène HBs.

Il est à remarquer que les taux de polypeptides exprimés atteignent un maximum 10 à 30 jours après la transfection, l'expression tendant ensuite à décliner, même si les cultures en cause se sont révélées dans chaque cas capables d'assurer une production du peptide immunogène sur une durée pouvant être de plusieurs mois.

Le but de la présente invention est de fournir des cultures cellulaires productrices d'un poly-

3

peptide immunogène ayant les propriétés de l'antigène HBs, les caractéristiques de production de l'antigène HBs par ces cellules, étant stabilisées de façon à assurer une production continue et en quantités non sujette à variations importantes pendant des durées prolongées, en tous cas supérieures à 6 mois.

L'invention découle de la découverte que des clones cellulaires particuliers peuvent être sélectionnés parmi les cultures de cellules Vero transformées dans les conditions sus-indiquées, qui présentent les caractéristiques souhaitées de production de l'antigène HBs, tout en présentant des caractéristiques d'innocuité tout à fait complètes les rendant aptes à être utilisés pour la production de principes vaccinants contre l'hépatite virale B.

Le procédé d'isolement des clones producteurs est basé sur la combinaison du test de détection de la préservation dans les cellules transformées des caractéristiques immunologiques spécifiques des cellules Vero non transformées, et sur un test de détection de la préservation dans les cellules transformées des sensibilités connues pour les cellules Vero non transformées à des agents pathogènes déterminés.

Avantageusement, le test de détection de la préservation des propriétés immunologiques des cellules Vero met en jeu des anticorps spécifiques anti-Vero (mettant en jeu par exemple un test d'immunofluorescence effectué avec un sérum polyclonal spécifique anti-Vero). Le test de détection des susdites sensibilités à des agents pathogènes met avantageusement en jeu la capacité de ces cellules d'être infectées par le poliovirus, par exemple dans les conditions décrites par Boon E.J. et Albrecht P., Journal of Virological Methods, 1983, vol. 6, 193-202.

La combinaison des tests qui précèdent conduit à l'isolement des cellules capables de produire un peptide immunogène vaccinant à l'égard du virus

4

de l'hépatite virale B, le taux de production de ce polypeptide immunogène étant à la fois élevé et constant pendant une durée d'au moins 6 mois.

La combinaison de ces tests démontre encore que les cellules transformées ont conservé toutes les caractéristiques de la population parentale Vero.

S'il en est besoin, un troisième test peut être mis en jeu pour détecter les clones qui doivent être retenus pour la production des polypeptides vaccinants. Ce dernier test repose sur la vérification de l'absence de tumorigénèse induite dans des souris, auxquelles ont été implantées au préalable de fortes quantités des cellules en cause, par exemple $10^6$ cellules par souris.

L'invention consiste encore dans un procédé de production d'un polypeptide ayant les propriétés immunogènes semblables à celles de l'antigène HBs, ce procédé étant caractérisé par la culture en continu des susdits clones isolés dans un milieu de culture approprié au développement des cellules Vero, et par le remplacement périodique du milieu de culture, le polypeptide immunogène produit étant ensuite chaque fois retiré du milieu de culture.

D'autres caractéristiques de l'invention apparaîtront encore au cours de la description qui suit d'un mode préféré de construction d'une souche productrice conforme à l'invention.

LE CLONE VC 10

Le clone VC 10 de cellules Vero a été utilisé comme population parentale pour l'établissement du clone GAR 1412 qui exprime l'HBs. Le clone VC 10 a été isolé en mai 1980 de la population parentale Vero provenant de l'American Type Culture Collection (ATCC CCL 81), reçue au laboratoire au niveau du 25ème passage. Le clone VC 10 a été obtenu au niveau du 140ème passage (15 passages au laboratoire) par dilution terminale en milieu Dulbecco 5 % et sérum de veau. Un lot de 10 ampoules a été préparé et conservé en azote liquide.

LES PLASMIDES UTILISES POUR LA TRANSFORMATION DES SUSDITES CELLULES

On a utilisé tant le plasmide pAG 66 mentionné plus haut (représenté de façon schématique dans la fig. unique) qu'un plasmide obtenu à partir de ce dernier, après délétion d'un fragment StuI de 145 paires de bases (pb) dans la région 3' non codante du gène HBs (plasmide pAG 141).

Le plasmide pAG 141 a été construit de la façon suivante à partir de pAG 66. Il est tout d'abord rappelé que le gène HBs contient quatre sites StuI aux positions 1206, 1401, 2325 et 2470, selon le système de numérotation de GALIBERT et al (1979), Nature, 281, 646-650.

La première opération a donc consisté dans la digestion complète de pAG 66 par l'enzyme de restriction StuI, la récupération du fragment le plus important contenant l'ADN de pAG 66 autre que le gène S, mais y inclus les parties proximale et distale de celui-ci vis-à-vis du site EcoRI du plasmide pAG 66, la ligation de ce fragment et du fragment de taille moyenne isolé par électrophorèse sur gel (ce fragment correspondant à la partie du gène S s'étendant entre les positions 1401 et 2325) pour former le plasmide pAG 140 intermédiaire de 8130 pb, puis la réinsertion dans ce plasmide du petit fragment correspondant à celui qui, dans le gène HBs, s'étend entre les positions 1206 et 1401, en amont du fragment 721-1645 vis-à-vis du site EcoRI. Le plasmide obtenu comportant 8320 pb a été dénommé pAG 141.

6

TRANSFECTION DES CELLULES VERO CLONE VC10 ET OBTENTION DU
CLONE GAR 1412

La transfection du plasmide pAG 141 dans les cellules
a été réalisée en présence d'ADN entraîneur de sperme de
saumon selon la technique au phosphate de calcium décrite
par GRAHAM et VAN DER EB (1973). Vingt-quatre heures
après la transfection, les cellules ont été mises en
présence de l'agent sélectif, l'antibiotique G-418. Un
mois après la transfection, des clones de cellules résistantes au G-418 ont été isolés et étudiés pour la production de l'antigène HBs. Le clone GAR 1412, qui produit
et excrète l'HBs, a été cloné une deuxième fois pour être
étudié de façon plus approfondie.

Au cours de nombreuses expériences réalisées avec
ou sans entraîneur, avec les plasmides pAG 66, pAG 141,
ou des dérivés de ceux-ci, plus de 270 clones ont été
isolés et étudiés. Il a été trouvé que 6 % d'entre eux
produisent et excrètent l'HBs, qui est décelé par radio-
immuno-essai (AUSRIA, ABBOTT).

CINETIQUE D'EXPRESSION DE L'ANTIGENE HBs PAR LE CLONE
GAR 1412

Les cellules GAR 1412 sont trypsinées et ensemencées
dans du milieu de Eagle modifié par Dulbecco contenant
10 % de tryptose phosphate et 10 % de sérum de veau normal aseptique. La production maximale d'HBs est obtenue
3 à 4 semaines après l'ensemencement des flacons. Plus
de 85 % de l'antigène est excrété. Lorsque le plateau
de production de l'antigène est atteint, le taux d'expression peut être maintenu sans trypsiner les cellules mais
en renouvelant le milieu de culture deux fois par semaine
avec le milieu mentionné ci-dessus. Un taux d'expression
de 1 microgramme d'antigène HBs par millilitre de surnageant et par 24 heures peut être maintenu dans ces conditions pendant au moins 6 mois. Le taux d'antigène a été

7

calculé en utilisant comme référence la solution référence à 20 microgrammes/millilitre de MERCK SHARP & DOHME.

CARACTERISATION DE L'ANTIGENE HBs EXCRETE PAR LE CLONE GAR 1412

L'antigène HBs produit et excrété par le clone GAR 1412 a la même densité en gradient de CsCl que l'antigène préparé à partir de plasma humain ou produit par certaines lignées d'hépatome humain. L'antigénicité de l'HBs produit par le clone GAR 1412 est mise en évidence par radio-immuno-essai. Des études d'immunomicroscopie électronique ont montré que l'antigène est porté par des particules sphériques de 22 nm environ de diamètre, semblables à celles que l'on isole du plasma humain.

STABILISATION DE L'EXPRESSION DE L'HBs PAR LE CLONE GAR 1412

La stabilité de l'expression de l'HBs par le clone GAR 1412 a été étudiée pendant plus de 7 mois, les cellules étant divisées dans un rapport de surface 1:12 une fois par semaine (100 générations cellulaires). La synthèse d'HBs s'est avérée stable sur cette période aussi bien en présence qu'en absence de sélection par le G 418. La synthèse maximale d'HBs n'est cependant obtenue qu'en laissant "vieillir" les cellules pendant 3 à 4 semaines après la trypsination comme décrit dans le paragraphe "Cinétique d'expression de l'antigène HBs par le clone GAR 1412".

TUMORIGENESE DES CLONES VC10 et GAR 1412

Les recommandations de l'Organisation Mondiale de la Santé concernant la préparation du vaccin antipoliomyélitique inactivé stipulent la possibilité d'employer comme substrat cellulaire les lignées cellulaires continues, à condition qu'elles s'avèrent non tumorigènes pour les animaux immunodéprimés (OMS Rapport Technique 673-Révision 1981, p. 76-82).

8

Des études effectuées par PETRICCIANI et Coll.
(J. Nat. Cancer Inst. 1973, 51, 191-196 et Develop. Biol.
Standard. 1981, 50, 27-35) ont montré que les lignées
continues de singe (parmi lesquelles les cellules Vero)
ne sont pas tumorigènes quand elles sont essayées dans
différents systèmes in vitro et in vivo. L'emploi de
telles cellules pour la production de vaccins viraux est
possible si le produit est purifié en vue de l'élimination
de l'acide nucléique (Intervirology, 1980, 14, 300-309
et Develop. Biol. Standard. 1981, 50, 59-69).

Pour examiner la possibilité d'employer le clone
GAR 1412 qui exprime d'une manière stable l'antigène HBs
pour la production d'un vaccin anti-hépatite B, la tumorigénèse des clones VC 10 et GAR 1412 a été testée. Dans
ce but, des cellules des clones VC 10 et GAR 1412 et de
la lignée HeLa ont été implantées chez 5 souris nu/nu
(pour chaque lignée) à raison de $10^6$ cellules par souris.
Après 21 jours, seuls les animaux inoculés avec les cellules HeLa ont développé des tumeurs, tandis que les souris ayant reçu les cellules VC 10 et GAR 1412 sont restées négatives. Ces résultats ont été confirmés par l'examen histologique.

CARACTERISATION DES CELLULES GAR 1412

Les cellules GAR 1412 qui expriment l'HBs se sont
avérées positives dans un test d'immunofluorescence effectué avec un sérum polyclonal spécifique anti-Vero et
elles restent sensibles au poliovirus. Ces résultats
montrent qu'il s'agit bien d'une cellule ayant les caractéristiques de la population parentale Vero.

PRODUCTION DE L'HBs

A partir du clone GAR 1412, il est possible d'effectuer des cultures en masse pour obtenir des quantités
importantes d'HBs qui peuvent être traitées ultérieurement
pour produire un vaccin anti-hépatite B.

Les cellules du clone GAR 1412 produisent et ex-

9

crètent continuellement l'Ag HBs (particule de 22 nm) qui n'est pas infectieux et qui n'a donc aucun effet sur la viabilité des cellules productrices. Lorsque le milieu de culture utilisé pour la production d'HBs est épuisé, il peut être retiré et remplacé par un milieu frais. Les cellules peuvent produire continuellement l'Ag HBs à condition d'utiliser un milieu qui maintient les cellules en vie. Les expériences menées jusqu'à présent ont montré que le changement régulier (1-2 fois par semaine) du milieu de culture permet un minimum de 6 mois de production continue de l'Ag HBs par la même culture cellulaire.

Une production à grande échelle de l'HBs sur cellules GAR 1412, en vue de la préparation d'un vaccin, est possible aussi bien dans des boîtes de Roux de 200 $cm^2$ de surface de culture que dans des appareils industriels de culture cellulaire comme celui commercialisé sous la désignation GYROPEN (Chemap, Mannedorf, Suisse), où dans d'autres systèmes de culture cellulaire en masse.

Le milieu de culture qui peut être constitué de milieu Dulbecco contenant 10 % de tryptose-phosphate et 5 % de sérum de veau est changé une à deux fois par semaine. Le milieu de culture dans lequel l'HBs a été libéré est récolté et utilisé comme source d'HBs pour la production du vaccin anti-hépatite B. L'HBs lui-même est alors recueilli, par exemple par ultracentrifugation en gradient continu de chlorure de coesium ou par passage sur une colonne d'affinité sur laquelle ont au préalable été fixés des anticorps anti-HBs.

POUVOIR IMMUNOGENE DE L'Ag HBs PRODUIT PAR LE CLONE GAR 1412

Le pouvoir immunogène de l'Ag HBs produit par les cellules GAR 1412 a été testé dans un essai recommandé par le National Institute for Biological Standardization and Control (NIBSC, Londres), en parallèle avec un vaccin anti-hépatite B du commerce, obtenu à partir de l'HBs humain. Des lots de 10 souris Balb C âgées de cinq semaines ont été immunisées par voie intrapéritonéale avec
1 ml de chaque dilution de chaque vaccin contenant 0,5 mg
d'hydroxyde d'aluminium par millilitre.

Les animaux ont été immunisés par une seule injection avec les deux vaccins selon le schéma suivant :

Quantité d'antigène :

| 4 microgrammes/ml | inoculés à chaque souris (10 souris) | | | | |
|---|---|---|---|---|---|
| 1 " " | " | " | " | " | " |
| 0,25 " " | " | " | " | " | " |
| 62 ng/ml | " | " | " | " | " |
| diluant | " | " | " | . " | (5 souris) |

(témoin négatif)

Vingt-huit jours après l'injection d'antigène, les
souris sont saignées et les sérums sont examinés individuellement pour la présence d'anticorps anti-HBs, en utilisant une trousse de diagnostic AUSAB (ABBOTT).

En calculant l'indice d'extinction antigénique (la
quantité minimale d'antigène capable d'induire des anticorps chez 50 % des animaux), nous avons trouvé les valeurs suivantes :

Ag HBs - GAR 1412      - 62 ng/ml
Vaccin commercial      - 65 ng/ml.

Ce résultat montre que le pouvoir immunogène de
l'HBs produit par le clone GAR 1412 est équivalent au
pouvoir immunisant du vaccin commercial.

Cette expérience indique que l'HBs exprimé par les
cellules GAR 1412 peut être utilisé comme matière première
dans la préparation d'un vaccin anti-hépatite B.

Le clone GAR 1412 a été déposé à la Collection
Nationale de Culture de Micro-Organismes (C.N.C.M.) de
l'INSTITUT PASTEUR de Paris, le 30 novembre 1983
sous le n° I-267.

Le tri des clones retenus au terme de l'opération décrite à la page 6 sous le sous-titre "Transfection des cellules Vero clone VC 10 et production du clone GAR 1412" a été réalisé comme suit :

Chaque clone est transféré à partir de la boîte de transfection, dans une boîte de 25 cm$^2$. Lorsque le clone a atteint la subconfluence, il est trypsinisé in situ pour étaler toutes les cellules. Puis un dosage radio-immunologique de détection de l'HBs est effectué au 7ème et au 15ème jours après la trypsination. Si le clone est capable au 15ème jour d'excréter des quantités d'HBs voisines de 50 ng/ml par 5 jours, il est conservé pendant 1 ou 2 mois. Il est alors également vérifié que les clones retenus ont conservé les propriétés immunologiques des cellules Vero et qu'ils restent également sensibles au poliovirus.

L'antigène produit par les cellules GAR 1412 s'est révélé comporter le récepteur pour l'albumine humaine polymérisée codée par le gène situé dans la région pré-S de DNA-HBV. Ces observations ont été faites à l'aide de réactions immunologiques appropriées. L'antigène codé par le clone GAR 1412 contient par conséquent l'un des épitopes majeurs intervenant dans la synthèse d'anticorps anti-HBs producteurs (NEURATH-Science, 224, 1984, 392).

Des clones satisfaisant à l'ensemble de ces conditions sont conformes à l'invention.

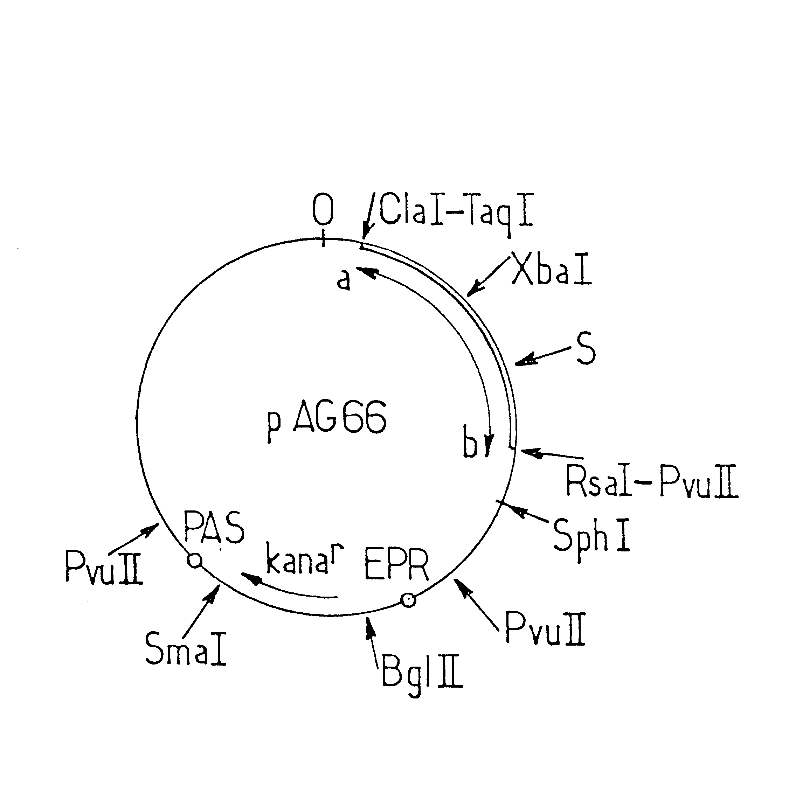

## 12
## REVENDICATIONS

1 - Cellules eucaryotes transformées de mammifères appropriées à la fabrication de vaccins, contenant, à titre de marqueur génétique interne sélectif,
un plasmide contenant un promoteur eucaryote ou reconnu
par sa capacité à diriger l'expression dans une cellule
eucaryote, de préférence d'un organisme de mammifère,
d'un gène qui lui est normalement associé, et une séquence d'ADN codant pour une enzyme susceptible d'inactiver l'antibiotique G 418, cette dernière séquence étant
liée au promoteur susdit et sous son contrôle direct, ce
plasmide contenant également encore une séquence d'ADN
codant pour le peptide immunogène particulier dont la
production est recherchée, caractérisées en ce que ces
cellules de mammifères sont constituées par des cellules
Vero, que la séquence codant pour le peptide immunogène
recherché est une séquence codant pour un peptide immunogène ayant les propriétés de l'antigène HBs, et en ce
que lesdites cellules ont conservé les propriétés immunologiques des cellules Vero non transformées et une
capacité d'être infectées par le poliovirus.

2 - Cellules transformées selon la revendication
1, caractérisées en ce qu'elles réagissent avec des anticorps polyclonaux formés contre des cellules Vero non
transformées.

3 - Cellules selon la revendication 1, caractérisées en ce qu'elles sont transformées par le plasmide
pAG66.

4 - Cellules selon la revendication 1, caractérisées en ce qu'elles sont transformées par le plasmide
pAG141.

5 - Cellules selon l'une quelconque des revendications 1 à 4, caractérisées en ce qu'elles ne sont pas
tumorigènes dans le test reposant sur la vérification de
l'absence de tumorigénèse induite dans des souris, aux-

0145589

13

quelles ont été implantées au préalable $10^6$ desdites cellules par souris.

6 - Procédé de production d'un polypeptide ayant les propriétés immunogènes semblables à celles de l'antigène HBs, caractérisé par la culture en continu des cellules selon l'une quelconque des revendications 1 à 5 dans un milieu de culture approprié au développement des cellules Vero, et par le remplacement périodique du milieu de culture, le polypeptide immunogène produit étant ensuite chaque fois retiré du milieu de culture.